# EUROPÄISCHE PATENTSCHRIFT

(11) **EP 2 132 161 B1**
(45) Veröffentlichungstag und Bekanntmachung des Hinweises auf die Patenterteilung: **10.04.2013**
(21) Anmeldenummer: 08709162.5
(22) Anmeldetag: 21.02.2008
(51) Int. Cl.: C07C 205/59, C07C 201/08

(54) **VERFAHREN ZUR NITRIERUNG SUBSTITUIERTER BENZOLE IN GEGENWART VON PROPIONSÄURE**
METHOD FOR THE NITRATION OF SUBSTITUTED BENZENES IN THE PRESENCE OF PROPIONIC ACID
PROCEDE DE NITRATION DE BENZENES SUBSTITUES EN PRESENCE D'ACIDE PROPIONIQUE

(30) Priorität: 02.03.2007 DE 102007010161
(43) Veröffentlichungstag der Anmeldung: 16.12.2009
(73) Patentinhaber: Saltigo GmbH, 40764 Langenfeld (DE)
(72) Erfinder: LANGER, Reinhard, 47918 Tönisvorst (DE); GOTTA, Matthias, 51061 Köln (DE); KISSENER, Wolfram, 53819 Neunkirchen-Seelscheid (DE); HÖLZER, Bettina, 29664 Walsrode (DE)
(74) Vertreter: Pettrich, Klaus-Günter
(86) Internationale Anmeldenummer: PCT/EP2008/052128
(87) Internationale Veröffentlichungsnummer: WO 2008/107312

(56) Entgegenhaltungen:
- EP-A- 1 323 705
- DE-A1- 19 917 524
- US-A- 5 488 187

## Beschreibung

Die vorliegende Erfindung betrifft die technische Nitrierung von substituierten Benzolen zur selektiven Herstellung von substituierten Nitrobenzolen.

Substituierte Nitrobenzole sind wichtige Zwischenprodukt für die Herstellung von Chemikalien, Pharmaprodukten und Pflanzenschutzmitteln.

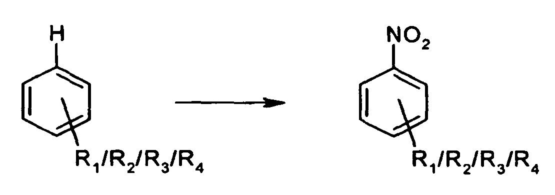

Leider ist die großtechnische Nitrierung von Aromaten mit verschiedenen Schwierigkeiten behaftet.

Die gewünschten Produkte sind in der Regel nicht so isomerenrein zugänglich, da das Isomerengleichgewicht bei der Mononitrierung verschieden substituierter Aromaten bekanntlich nur wenig von den Reaktionsbedingungen beeinflussbar ist (siehe Mononitrierung von Toluol: K. Weissermehl, H.-J. Arpe, Industrielle organische Chemie, 3. Auflage, Seite 400-401) und die Reinisolierung der Regioisomere ist schwierig. Ferner ist das Nitrieren mit hochkonzentrierten Salpetersäuren sicherheitstechnisch nicht unbedenklich.

Um die Selektivität positiv zu beeinflussen wird daher oft bei niedrigen Temperaturen in Gegenwart von organischen Lösungsmitteln und bei bestimmten Wassergehalten nitriert.

So beschreibt US 2003/0191341, als das vorerst letzte Patent einer ganzen Reihe, die Nitrierung von substituierten Diphenylethern mit Salpetersäure oder Gemischen aus Salpetersäure und Schwefelsäure in chlorierten Kohlenwasserstoffen bei -10 bis +10°C in Gegenwart bestimmter Mengen an Acetanhydrid. Nachteilig ist an dem Verfahren, das ein schlecht rührbarer Slurry entsteht und dass das chlorierte Lösungsmittel zu entsorgen ist.

DE-A 199 17 524 (Boehringer, 17.4.1999) beschreibt die Nitrierung von 4-Alkaloylamino-3-alkyl-benzoesäurealkylestern gelöst in einem Gemisch aus Schwefelsäure, Salpetersäure und Wasser, zu dem in einem weiteren Schritt konzentrierte Salpetersäure bei -5 bis +5°C dosiert wird. Es entsteht mit 91%iger Ausbeute praktisch isomerenfreies Produkt. Durch Vorlegen der Nitriersäuremischung und Dosierung des Eduktes oder Vorlegen des Eduktes in wasserarmer Schwefelsäure und Dosierung der Salpetersäure-Schwefelsäure-Mischung werden schlechtere Ergebnisse erzielt. Es entsteht dann Produkt mit 8-10% 6-Nitro- und 1-2% 2-Nitro-Isomer. Pro mol Edukt müssen 1,8-2,2 mol Salpetersäure, 1,8-2,2 mol Schwefelsäure und 2,5-4 mol Wasser als Lösungsmittel zugegen sein, dann wird durch Zugeben von 7-9 mol Salpetersäure nitriert. Gemische aus Schwefelsäure und Salpetersäure können nicht wirtschaftlich zugesetzt werden, jedenfalls wird das nicht beansprucht. Nachteil des Verfahrens ist die niedrige Temperatur und der offenbar enge Bereich in dem die Umsetzung selektiv verläuft.

DE-A 39 17 733 beschreibt die Nitrierung von 2,2-Bis(4-hydroxyphenyl)hexafluoropropan in Essigsäure mit wasserhaltigen hochkonzentrierten Salpetersäuren bei 30 bis 70°C. Das Lösungsmittel Essigsäure wird hier gewählt, weil nach der Reaktion durch Zugabe von Wasser das Produkt in hoher Reinheit auskristallisiert und das Zwischenprodukt in Lösung bleibt. Es werden als Lösungsmittel auch die höheren Alkansäuren, genannt niedere Fettsäuren, mit bis zu 5 Kohlenstoffatomen im Claim genannt, allerdings sind nur Versuche mit Essigsäure im Experimentellen Teil. Ob die Carbonsäuren als Lösungsmittel für die Nitrierung günstige Eigenschaften haben, sich diese unterscheiden und höhere Carbonsäuren besser oder schlechter sind bleibt offen.

Aufgabe der Erfindung war es, ein breit anwendbares Verfahren zur Nitrierung von substituierten Benzolen aufzufinden, bei dem ein gut abbaubares und billiges organisches Lösungsmittel eingesetzt wird und das außergewöhnlich günstige Eigenschaften bezüglich Selektivität, Reaktionstemperatur und Sicherheit bietet.

Gegenstand der Erfindung ist daher die Bereitstellung eines breitanwendbaren Verfahrens zur Nitrierung von substituierten Benzolen.

Überraschenderweise wurde gefunden, dass die gesuchten Verfahrenseigenschaften erzielt werden, wenn man substituierte Benzole der Formel 1 in Propionsäure gelöst zur Reaktion bringt.

Gegenstand der Erfindung ist somit ein Verfahren zur Nitrierung von substituierten Benzolen der allgemeinen Formel 1 zu Nitrobenzolen der allgemeinen Formel 2 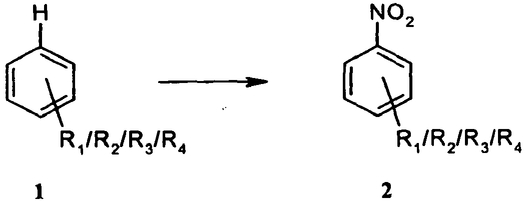 worin R₁, R₂, R₃ und R₄ gleich oder verschieden sein können und für -H, -OR', -NHR", -Alkyl, -Cycloalkyl, -Aryl, -Halogen, -Trihalogenmethyl, -COOR', -COR' und -CN stehen und R' für -H, - Alkyl, -Aryl steht, mit den gleichen möglichen Substitutenten wie R₁-R₄, und R" für -COR' steht, mit hochkonzentrierter Salpetersäure oder einer Mischung aus Salpetersäure und Schwefelsäure, dadurch gekennzeichnet, dass das substituierte Benzol in Gegenwart von 50 bis 5000 Gew%, (rel. zum Edukt) Propionsäure, 1 bis 20 Gew% (rel. zum Edukt) Schwefelsäure und 10 bis 100 Gew.-% (rel. zum Edukt) einer wasserbindenden Substanz aus der Gruppe Propionsäureanhydrid, Essigsäureanhydrid, P₂O₅ und SO₃ nitriert wird.

Zur Nitrierung kann hochkonzentrierte Salpetersäure eingesetzt werden, bevorzugt wird eine Mischung aus Salpetersäure und Schwefelsäure eingesetzt.

Die Reaktionstemperatur liegt zwischen -10 und 50°C, bevorzugt zwischen 0 und 40°C.

Als Reaktoren sind alle für die Umsetzung flüssiger Verbindungen geeigneten Reaktoren denkbar, wie z.B. gerührte Kessel oder Verweilzeitrohre.

Das Verfahren kann batchweise oder kontinuierlich betrieben werden.

Bisher war noch kein Verfahren bekannt, bei dem Propionsäure als Lösungsmittel für Nitrierungen eingesetzt wurde. Im Vergleich zum Stand der Technik ist das erfindungsgemäße Verfahren sehr günstig in Bezug auf Selektivität, Reaktionstemperatur und Sicherheit.

Im Folgenden wird das erfindungsgemäße Verfahren anhand einiger Beispiele illustriert, wobei die Beispiele jedoch nicht als Einschränkung des Erfindungsgedankens zu verstehen sind.

### Beispiele:

### Beispiel 1 (Acifluorfen)

40 g 3-(Chlor-4-trifluormethyl-phenoxy)-benzoesäure werden in 75 g Propionsäure bei 20°C suspendiert und mit 35,9 g Propionsäureanhydrid versetzt. Die Suspension wird rasch zu einer trüben Lösung mit wenig Feststoff. Es werden 3,6 g Schwefelsäure zugesetzt, worauf die Temperatur auf 36°C steigt und eine dunkel rot-braune Lösung entsteht, diese wird auf 1-2°C abgekühlt. Nun wird in 2 h 8,8 g konzentrierte Salpetersäure zudosiert und 1 h nachgerührt. Dann wurden nochmals 0,37 g Salpetersäure zugesetzt und 1 h bei 10°C nachgerührt.

Das Reaktionsgemisch wird in 194,3 g siedendes Wasser ausgetragen. Es bildet sich eine braune klare Lösung, die in 45 Minuten auf 5°C abgekühlt wird. Der Niederschlag wird abgesaugt und 3 mal mit 48,6 g Wasser gewaschen.

Man erhält 42,2 g feuchten Feststoff, der im Vakkumtrockenschrank zu 35,4 g trockenen Feststoff umgewandelt wird. Mit 76,1%iger Ausbeute wird Acifluorfen erhalten, dass auf 100 Teile Wirkstoff 5 Teile 2-Nitro- und 0,7 Teile 6-Nitro-Isomeres enthält. Es sind weniger als 1% Dinitroderivate entstanden. Die Molbilanz weist 89,5% Acifluorfen, 6,5% 2-Nitro- und 3% 6-Nitro-Isomer aus.

### Beispiel 2 (Acifluorfen mit Mischsäure GTM 1266)

100 g 3-(Chlor-4-trifluormethyl-phenoxy)-benzoesäure werden in 187,5 g Propionsäure bei 20°C suspendiert und mit 55,4 g Propionsäureanhydrid versetzt. Es werden 9,4 g Schwefelsäure zugesetzt und die Lösung auf 0°C abgekühlt. Nun wird 71,6 g 33,3%ige Mischsäure (Salpetersäure gelöst in Schwefelsäure) in 1,5 h zudosiert und 1 h bei 0°C und 1 h bei 10°C nachgerührt.

Die Analyse des Gemisches zeigt, dass zu 89mol% Acifluorfen, zu 6,8mol% 2-Nitro- und zu 2,6mol% 6-Nitro-isomer entstanden sind. Es sind weniger als 1% Dinitroderivate entstanden.

### Beispiel 3 (NiBAMBE / 2531-15)

150 g BAMBE (4-Butanoylamino-3-methyl-benzosäuremethylester) 281 g Propionsäure, 76,8 g Essigsäureanhydrid und 18,6 g Schwefelsäure werden zusammen auf 30°C erwärmt und langsam in 5 h bei maximal 32°C mit 153 g 33,3%iger Mischsäure versetzt. Bei der Mischsäurezugabe geht alles vollständig in Lösung und hellt sich auf. Es wird 1 h bei 30°C nachgerührt. Es werden 1200 g Wasser vorgelegt und bei 5-10°C mit dem Reaktionsansatz verrührt. Der Niederschlag wird abgesaugt und 2 mal mit 400 g Wasser gewaschen.

Es werden 285 g hell gelber feuchter Feststoff erhalten, der 158,5 g NiBAMBe (4-Butanoylamino-3-methyl-5-nitro-benzoesäuremethylester) 100% enthält, was einer Ausbeute von 88,7% entspricht. Neben 96,7% NiBAMBE werden 0,51% BAMBE und 0,4% Isomere gefunden.

## Patentansprüche

1. Ein Verfahren zur Nitrierung von substituierten Benzolen der allgemeinen Formel 1 zu Nitrobenzolen der allgemeinen Formel 2 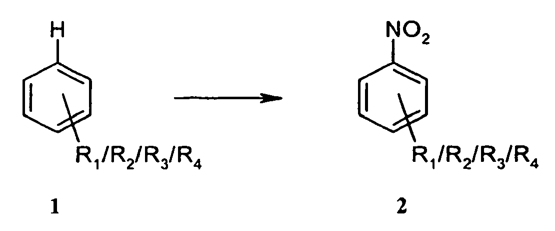 worin R₁, R₂, R₃ und R₄ gleich oder verschieden sein können und für -H, -OR', -NHR", -Alkyl, -Cycloalkyl, -Aryl, -Halogen, -Trihalogenmethyl, -COOR', -COR' und -CN stehen und R'für -H, -Alkyl, -Aryl steht, mit den gleichen möglichen Substitutenten wie R₁-R₄, und R" für -COR' steht, mit hochkonzentrierter Salpetersäure oder einer Mischung aus Salpetersäure und Schwefelsäure, **dadurch gekennzeichnet, dass** das substituierte Benzol in Gegenwart von 50 bis 5000 Gew%, (rel. zum Edukt) Propionsäure, 1 bis 20 Gew% (rel. zum Edukt) Schwefelsäure und 10 bis 100 Gew.-% (rel. zum Edukt) einer wasserbindenden Substanz aus der Gruppe Propionsäureanhydrid, Essigsäureanhydrid, P₂O₅ und SO₃ nitriert wird.

2. Verfahren nach Anspruch 1, **dadurch gekennzeichnet, dass** 100 bis 300 Gew.-% (rel. zum Edukt) Propionsäure eingesetzt werden.

3. Verfahren nach Anspruch 1 bis 2, **dadurch gekennzeichnet, dass** die Reaktionstemperatur zwischen -10 und 50°C liegt.

4. Verfahren nach Anspruch 1 bis 3, **dadurch gekennzeichnet, dass** die Reaktionstemperatur zwischen 0 und 40°C liegt.

5. Verfahren nach Anspruch 1, **dadurch gekennzeichnet, dass** die wasserbindende Substanz Propionsäureanhydrid oder Essigsäureanhydrid ist.

## Claims

1. Process for nitrating substituted benzenes of the general formula 1 to nitrobenzenes of the general formula 2 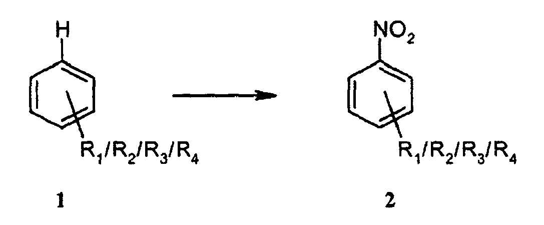 in which R₁, R₂, R₃ and R₄ may be the same or different and are each -H, -OR', -NHR", -alkyl, -cycloalkyl, -aryl, -halogen, -trihalomethyl, -COOR', -COR' and -CN, and R' is -H, -alkyl, -aryl, with the same possible substituents as R₁-R₄, and R" is -COR', with highly concentrated nitric acid or a mixture of nitric acid and sulphuric acid, **characterized in that** the substituted benzene is nitrated in the presence of 50 to 5000% by weight (relative to the reactant) of propionic acid, 1 to 20% by weight (relative to the reactant) of sulphuric acid and 10 to 100% by weight (relative to the reactant) of a water-binding substance from the group of propionic anhydride, acetic anhydride, P₂O₅ and SO₃.

2. Process according to Claim 1, **characterized in that** 100 to 300% by weight (relative to the reactant) of propionic acid is used.

3. Process according to Claims 1 and 2, **characterized in that** the reaction temperature is between -10 and 50°C.

4. Process according to Claims 1 to 3, **characterized in that** the reaction temperature is between 0 and 40°C.

5. Process according to Claim 1, **characterized in that** the water-binding substance is propionic anhydride or acetic anhydride.

## Revendications

1. Procédé de nitration de benzènes substitués de formule générale 1 en nitrobenzènes de formule générale 2 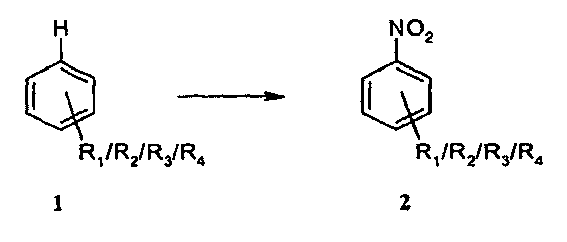 dans lesquelles R₁, R₂, R₃ et R₄ peuvent être identiques ou différents, et représentent -H, -OR', -NHR", - alkyle, -cycloalkyle, -aryle, -halogène, - trihalogénométhyle, -COOR', -COR' et -CN, et R' représente -H, -alkyle, -aryle, avec les mêmes substituants possibles que R₁ à R₄, et R" représente - COR', avec de l'acide nitrique hautement concentré ou un mélange d'acide nitrique et d'acide sulfurique, **caractérisé en ce que** le benzène substitué est nitré en présence de 50 à 5 000 % en poids (par rapport au réactif) d'acide propionique, 1 à 20 % en poids (par rapport au réactif) d'acide sulfurique et 10 à 100 % en poids (par rapport au réactif) d'une substance se liant à l'eau du groupe constitué par l'anhydride de l'acide propionique, l'anhydride de l'acide acétique, P₂O₅ et SO₃.

2. Procédé selon la revendication 1, **caractérisé en ce que** 100 à 300 % en poids (par rapport au réactif) d'acide propionique sont utilisés.

3. Procédé selon la revendication 1 ou 2, **caractérisé en ce que** la température de réaction se situe entre -10 et 50 °C.

4. Procédé selon les revendications 1 à 3, **caractérisé en ce que** la température de réaction se situe entre 0 et 40 °C.

5. Procédé selon la revendication 1, **caractérisé en ce que** la substance se liant à l'eau est l'anhydride de l'acide propionique ou l'anhydride de l'acide acétique.
